# EUROPEAN PATENT APPLICATION

(11) **EP 1 547 580 A1**
(43) Date of publication of application: **29.06.2005**
(21) Application number: 03029799.8
(22) Date of filing: 23.12.2003
(51) Int. Cl.: A61K 9/127

(54) **Loading of a camptothecin drug into colloidal nanoparticles**

(71) Applicant: MediGene Oncology GmbH, 82152 Planegg/Martinsried (DE)
(72) Inventor: The designation of the inventor has not yet been filed
(74) Representative: Weiss, Wolfgang, Dipl.-Chem. Dr.

(57) **Abstract**

The present invention relates to an improved method of producing a colloidal nanoparticulate preparation comprising a camptothecin drug in its carboxylate form, a kit and a pharmaceutical composition.

## Description

The present invention relates to an improved method of producing a colloidal nanoparticulate preparation comprising a camptothecin drug in its carboxylate form, a kit for the preparation of said preparation and a pharmaceutical composition thereof.

Camptothecin (CPT) is a quinoline-based alkaloid, which can be isolated from the Chinese tree Camptotheca acuminata (Wall, Wani et al. 1966). It was first described and tested as an anti-cancer drug in the 60ies and 70ies. Anti-tumor activity was noted in animal models and in clinical studies. However, patients experienced severe side reactions such as neutropenia, thrombocytopenia, haemorrhagic cystitis (1). The therapeutic effect of camptothecin in humans had been questioned (Moertel, Schutt et al. 1972; Muggia, Creaven et al. 1972). It continued to be of high interest as a potential candidate for the development of an anti-cancer drug, and it was found that it has a particular mode of action, wherein binding to the topoisomerase I-DNA complex induces DNA breaks and cell death (topoisomerase I inhibitor) (2).

A fundamental molecular property of CPT is its pH dependent equilibrium between the lactone and the carboxylate form. The lactone form is lipophilic, while the carboxylate, which predominates at physiological pH and above, is water-soluble. Since the lactone form is too lipophilic to be administered without difficulties, initially, CPT was transformed into the water-soluble sodium salt (NCS 100880). However, due to unacceptable side reactions the development of that compound was not further pursued (Moertel, Schutt et al. 1972) (Muggia, Creaven et al. 1972).

In subsequent research, the equilibrium between the lactone form and the carboxylate form was found fundamental for the cytostatic activity and the appearance of side effects within anti-cancer treatment: CPT-carboxylate was identified as being responsible for the observed side reactions and it was considered to be significantly less active than CPT-lactone (Hertzberg, et. al. 1989).

Due to these futile properties of CPT-carboxylate, further efforts for the development of CPT based drugs concentrated on the control of the equilibrium between the lactone and the carboxylate form. A main objective of the development of CPT drugs was to stabilize the lactone form and to find ways to administer it without difficulties (Zunino et al. 2002). In a lager number of attempts, chemical functionalization has been performed in order to obtain CPT derivates or pro-drugs, which are water soluble and stable in the lactone form under physiological conditions.

In another approach, liposomes were used to protect CPT-lactone from hydrolysis. Liposomes play a significant role in medical and pharmaceutical sciences as drug delivery systems. Typically, an active compound, if it is lipopohilic, is embedded in the bilayer lipid membrane of the liposome or, if the compound is hydrophilic, it is encapsulated into the aqueous compartment. For the preparation and drug loading of liposomes a variety of well-known methods is available (R.R. C. New (ed.) Liposomes, A Practical Approach, Oxford University Press, Oxford 1990).
In (US 5552156), by embedding CPT-lactone in the hydrophobic region of the vesicular lipid bilayer, the lactone form was less exposed to the aqueous environment and hydrolysis was significantly slowed down. However, only very low drug/lipid ratios could be achieved and therefore the necessary dosages for clinical use could not be realized.
In a further liposome-based approach, the hydrophobic CPT-lactone was embedded into the lipid bilayer of a liposome comprising phospholipids, which contain unsaturated fatty acids (US 5834012). Thereby a stabilization effect was reported. It was proposed that the latter was due to the interaction of CPT in the lactone form with the unsaturated fatty acid chains of the lipids.

Water-soluble compounds can be encapsulated in the aqueous compartment of a liposome by forming the lipid vesicles in the aqueous solution of the compound (passive loading). However, this has the disadvantage that most of the compound remains in the free aqueous phase and usually it needs to be removed by dialysis. A variety of methods has been described to overcome this intrinsic problem of low encapsulation efficacy into the aqueous compartment of liposomes. One of it is the active loading technique, which is applicable to compounds where the membrane permeability can be different, for example as a function of the pH value. In that case, it the molecular properties of a molecule changes as a function of the pH in an appropriate way, by applying a pH gradient from the inner to the other side of the liposome, the compound can be trapped in the vesicle.

Encapsulation in the liposomal aqueous compartment of CPT drugs which were water-soluble or water-insoluble in the lactone form was realized by passive and by active loading (Burke and Gao, 1994, Emerson et al. 2001, Liu et al. 2002) to protect the lactone from hydrolysis and to enhance anti-tumor efficacy. However, up to now no substantial breakthrough towards a functional CPT drug formulation for practical applications could be achieved.

Another way to load liposomes with an active compound is disclosed in WO 96/05808 and WO 99/49716. Therein a method for producing concentrated 'vesicular phospholipid gels' by using high-pressure homogenisation is described. These semi-solid phospholipid pastes or -gels with high lipid content consist predominantly of vesicular structures (WO 96/05808, WO 99//49716 and Brandl 2001 (M. Brandl (2001) Liposomes as drug carriers: a technological approach, Biotechnology annual review Volume 759-85). It is reported to form liposome suspensions after dilution. WO 96/05808 discloses liposome preparations from unilamellar vesicles of small and medium size, with high/drug ratios of at least 20 % w/w. However, several disadvantages are linked to that approach: The preparation is highly viscous, and redispersion is done best under rigorous mechanical stress, such as an oscillating bath mill, which is a disadvantage for delicate materials. Storage of the active compound and the lipid fraction together is impeded if one of the components causes degradation of the other. This is particularly critical since the components are present at high concentration. In this context WO 99/49716 refers to liposome gels, with at least 20 % of an active compound, wherein the compound is added to the liposome gel and, by heating or mechanical stress, the compound is equally distributed inside and outside the vesicles. However, due to the high viscosity of these liposome gels, and due to the size of the vesicles, sterile filtration, which is an important step during the formation of pharmaceutical preparations, is not possible. Also the approach is limited to particular lipid and drug combinations.

None of the described methods provided a substantial general breakthrough for the production of liposomal formulations, particularly cationic liposomal formulations of camptothecin. This is the more important since it was reported recently, that cationic liposomes have high affinity to angiogenic blood vessels around a solid tumor (Schmitt-Sody M. et al. (2003) Clin Cancer Res 9, 2335-41), which makes them useful for specific targeting of a drug to the tumor site (vascular targeting).

Stable loading of camptothecin into colloidal nanoparticles is further difficult since the requirement for good solubility of CPT in an aqueous medium is pH dependent, that is that the pH is sufficiently high (basic). These conditions are futile however for lipid stability and may cause lipid degradation. Thus, producing colloidal nanoparticles loaded with camptothecin is difficult to achieve, since both components require opposing conditions for stability in an aqueous environment. This is especially true since for practical pharmaceutical applications a sufficient chemical and physical stability during storage (shelf life) and before application to a patient (in use stability) is a necessary requirement.

Thus, the problem underlying the present invention is to provide a method for the preparation of cationic nanoparticles comprising camptothecin with a high drug to lipid ratio, sufficient chemical and physical stability for pharmaceutical application and high phamaceutical efficacy.

The solution to the above problem is achieved according to the invention by providing the embodiments characterized in the claims. The invention relates to a method of producing a colloidal preparation comprising cationic colloidal nanoparticles and a camptothecin drug in its carboxylate form, wherein said preparation is substantially free of camptothecin lactone, comprising the steps of
a) providing a camptothecin drug in its carboxylate form,
b) providing drug-free cationic colloidal nanoparticles and
c) incubating said camptothecin drug of step a) with the drug-free cationic colloidal nanoparticles of the step b) in an aqueous solution for a period of time sufficient to cause loading of said camptothecin carboxylate drug into said cationic colloidal nanoparticles without further steps.

The camptothecin carboxylate drug in step a) can be prepared by exposing the CPT drug to an alkaline environment, preferably at a pH above 9. It can be provided either as an aqueous solution (liquid or frozen) or as a dry product (dry salt, dehydrated and the like).
CPT-carboxylate can be obtained quantitatively from the lactone form of CPT by incubation the latter with at least an equimolar amount or an excess of base (e.g. NaOH or NH₄OH). In the most simple approach CPT lactone is stirred with 1 M NaOH or NH₄OH ovenight. Higher concentrations are favourable to accelerate the process. Thereby no indication for chemical degradation of the camptothecin carboxylate within a time scale of one month at a pH of about 14 can be found.
In a preferred embodiment of the present invention the CPT lactone is quantitatively converted into its water-soluble carboxylate form by mixing CPT lactone with an aqueous NaOH solution. The molar ratio of CPT/NaOH is preferably between about 1:1.7 to about 1:0.6, more preferably between about 1:1.4 and about 1:0.9 and most preferably between about 1:1.2 and 1:1. The CPT lactone/NaOH mixture is stirred for a certain period of time (between about 1 hour up to about 24 hours) at a temperature between about 0°C and about 100°C, more preferably between about 20°C and about 80°C and most preferably between about 25°C and about 60°C to allow complete CPT-carboxylate formation. The content of CPT lactone in the final mixture is preferably less than about 6% (molar ratio), more preferably less than about 3% and most preferably less than 2%. The stability of the CPT-Na solution at 4°C is preferably longer than about 1 h, more preferably longer than about 4 h and most preferably longer than about 24 h.

For loading a CPT carboxylate drug into cationic nanoparticles, a high partition coefficient of the drug into the nanoparticle in an aquous solution is essential. Attractive molecular interactions between drug and nanoparticles are favourable in order to provide a high partition coefficient.

Any other active agent with such molecular properties can be loaded into cationic nanoparticles in a similar way. The agent should be sufficiently water-soluble. Preferably, it should be an organic molecule which comprises an anionic moiety and a moiety which may interact by amphipatic interactions (e. g. aliphatic or aromatic hydrocarbons). The electrostatic interactions are favourable for loading, but are not the only driving force and are not sufficient for loading: simple anionic ions for example (Cl⁻, SO₄⁻) are not loaded into the nanoparticles.

Thus, it is a further object of the present invention to provide a method of producing a colloidal preparation comprising cationic colloidal nanoparticles and an active agent, comprising the steps of
a) providing an active agent,
b) providing drug free cationic nanoparticles and
c) incubating said active agent of step a) with the agent free cationic colloidal nanoparticles of step b) in an aqueous medium for a period of time sufficient to cause loading of said agent into said cationic nanoparticles without further steps.

Examples of active agents are drugs, pro-drugs or diagnostic agents. A suitable agent should be soluble in water at least up to the desired concentration in the final preparation for application, it should comprise an anionic molecular moiety and it should be able to at lease partially penetrate into a membrane or associate to the latter. The agent can also be derivatized or functionalized to optimize its molecular properties.
Examples for anionic groups in a molecule are sulfonic acids, carboxy groups, phosphatidic acids or alcohols. Examples for moieties which can facilitate penetration in the hydrophobic part of a nanoparticle are hydrocarbons, such as alkyl and aryl groups.
Anionic and amphoteric tensides are an example for a suitable type of molecules: they comprise an anionic or bipolar head group and a hydrophobic moiety which is short enough to provide solubility in water, but is sufficiently long to facilitate penetration in the hydrophopic compartment of a membrane. Further examples are short chain fatty acids, alkylsulfonates, alkylarylsulfonates, alkylpolyglycoethersulfonates, or alkyphenylpolyglycoethersulfonates. In the same way phosphatic acid esters are suitable. By derivatization of a molecule which, by itself does not have a sufficient partition coefficient in a cationic nanoparticle, a compound can be obtained which is suitable for loading.
In a preferred embodiment of the present invention the active agent may be modified with a moiety which has a high partition coefficient in the cationic nanoparticle. Modifying therein comprises covalently linking a negatively charged moiety to said compound, e. g. by an ester, thioester, ether, thioether, amide, amine, carbon-carbon bond or a Schiff Base, chelating said compound by a negatively charged ligand or encarcerating said compound within a negatively charged moiety such as a carcerand, calixarene, fullerene, crown or anti-crown ether.
Suitable diagnostics can be fluorescent dyes, which comprise a negatively charged moiety, such as fluoresceins, rhodamines, and related compounds. Other suitable diagnostic compound can be ion chelators used for example as MRI contrast agents. Depending on their molecular properties, they may be used directly or after functionalization.

Empty cationic colloidal nanonoparticles, that is without an agent or drug, can be prepared by methods well known in the art. They may be present in form of liposomes, micelles, emulsions, nanocapsules or any other type of nanoparticles. Colloidal nanoparticles may also be prepared from a concentrated vesicular or non-vesicular phase. The nanoparticles may be present as an aqueous dispersion (liquid formulations, e. g. obtained by reconstitution of a lyophilisate, or frozen) or as a solid product (e. g. as a lyophilisate). The latter can be dehydrated to a liquid formulation by adding an aqueous medium.

Cationic colloidal nanoparticles, preferably liposomes, can be formed by techniques well known in the art, for example via a lipid film or by an infusion procedure or a by mechanical dispersion technique. The lipid film procedure thereby comprises the steps of providing a thin lipid film by evaporation the solvent from organic solution of the lipid and suspending said lipid film in an aqueous solution.
The infusion procedure comprises the steps of adding an organic solution comprising the lipid where the organic solvent is preferebly water-soluble and/or volatile, to an aqueous solution. The mechanical dispersion techniques may comprise homogenization, high-pressure homogenization, extrusion, compounding, mechanical mixing or sonication.

The liposomes may be monodisperse and monolamellar as obtained by extrusion through membranes of defined pore size. In that case the size range is favourably between 50 and 500 nm, more favourably between 100 and 300 nm. They may have been sterile filtrated afterwards. The liposomes may also be polydisperse and optionally multilamellar in the size range of 10-2000 nm.

Step c) of the inventive method is performed by exposing the components of step a) and step b) to each other in an aqueous medium. This may be achieved by mixing an aqueous medium comprising the camptotehcin carboxylate (e. g. a thawed frozen solution or a reconstituted solid product such as a lyophilisate) with the liposome dispersion (liquid formulation or reconstituted from its dry precursor state such as a lyophilisate), or by adding the aqueous solution of the camptothecin carboxylate to the dry precursor of the aqueous liposome dispersion (3), or by adding the liposome dispersion to dry camptothecin carboxylate (as a solid product). Mixing can be performed between about 10 min and about 6 hours, preferably between about 30 min and about 2 hours at an incubation temperature of between about 4°C and about 25°C, preferably about 25°C.
Step c) is performed without any further steps (such as vigourous stirring or extrusion or any other mechanically stressful step) since loading of the cationic nanoparticles is a self assembly process.

A ready to use preparation is obtained either directly by mixing of the two components of step a) and b) of the inventive method, or may be obtained by further diluting said components before application to a patient. Optionally, further additives my be added such as pH active agents. Different ionic and pH conditions may be present in the camptothecin drug (see step a) and the nanoparticles (see step b). Favourably, the camptothecin carboxylate solution has a pH above 7.5, more favourably above 8. A favourable pH of colloidal nanoparticles is pH lower than 7.5, more favourably lower than 7. Both components may also comprise further pH active components (acids, bases, salts, buffers), as well as stabilizing agents (tocopherol, ascorbic acid, sugars, cryoprotectants, salts and the like).

The invention relates to an improved method for loading cationic nanoparticles with an active agent. Further, it relates to the use of a camptothecin drug in the carboxylate form for the preparation of loaded cationic colloidal nanoparticles in an aqueous suspension. Thereby the colloidal nanoparticles comprise at least one cationic amphiphile in addition to the camptothecin drug. Liposomes are a typical representative of colloidal nanoparticles. The novel approach is different to passive and active loading of nanoparticles, particularly liposomes. Exposing of an aqueous solution of camptothecin carboxylate to suspension of colloidal nanoparticles or lyophilized colloidal nanoparticles such as liposomes is sufficient to achieve loading of the latter. No further requirements or preparation steps are needed. No vigorous stirring, homogenisation, heating or an other effort is necessary for loading. A preparation with new particular properties is obtained, different to those of the two components before mixing. In particular, the preparation is characterized by improved pharmacological activity with respect to the individual components. The inventive method is applicaple also to other active agents with suitable molecular properties (water soluble, sufficient partition coefficient in the nanoparticle).

The single components of the preparation (such is an active agent, e. g. a camptothecin carboxylate drug and cationic colloidal nanoparticles) can thereby be produced and stored separately, in order to obtain a ready-to-use preparation directly before an application to a patient (kit).

Unless defined otherwise, all technical and scientific terms used in this specification shall have the same meaning as commonly understood by persons of ordinary skill in the art to which the present invention pertains.

"About" in the context of amount values refers to an average deviation of maximum +/- 30 %, preferably +/- 20 % based on the indicated value. For example, an amount of about 30 mol% cationic lipid refers to 30 mol% +/- 9 mol% and preferably 20 mol% +/- 6 mol% cationic lipid with respect to the total lipid/amphiphile molarity.

"Active agent" refers to therapeutically or diagnostically active agents such as drugs or imaging agents, dyes or fluorescent markers including proteins and peptide drugs etc. The present invention can also be used for any chemical compound or material that is desired to be applied in cationic colloidal nanoparticles and which is a water soluble organic molecule which comprises an anionic moiety and a moiety which may interact by amphipatic interactions.

"Amphiphile" refers to a molecule, which consists of a water-soluble (hydrophilic) and an oil-soluble (lipophilic) part. Lipids and phospholipids are the most common representatives of amphiphiles. In the text, lipid and amphiphile are used synonymously.

"Angiogenesis associated condition" e. g. refers to different types of cancer, chronic inflammatory diseases, rheumatoid arthritis, dermatitis, psoriasis, wound healing and others.

"Camptothecin" refers to 20(S)-Camptothecine (1H-Pyrano[3',4':6,7]indolizino[1,2-b]quinoline-3,14 (4H,12H)-dione, 4-ethyl-4-hydroxy-, (S)- ), **CAS 7689-03-4**. 'Camptothecin' or 'camptothecin drug' in the present context includes as well the carboxylate form of the drug.

"Camptothecin drug" refers to camptothecin itself or a derivative thereof. "Camptothecin carboxylate drug" refers to a camptothecin drug wihich is in its carboxylate form. A camptothecin derivative is obtained by any chemical derivatization of camptothecin (see structure). A non-limiting list of possible camptothecin drugs is given under:
http://dtp.nci.nih.gov as from Aug. 19, 2002. In the sketch of the molecule, the most frequent derivatization sites are outlined as R₁-R₅.

Structure of camptothecin drugs:

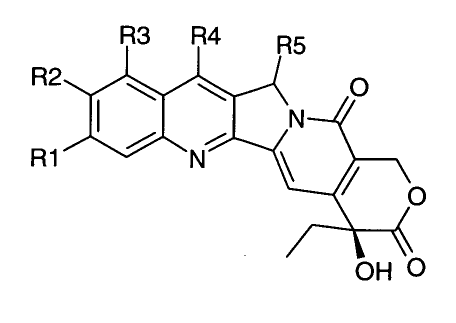

In Table 1, typical examples for derivatization at different sites are listed. Camptothecin may be present as a hydrochloride. The lactone ring (E-ring) may be seven-membered instead of six-membered (homocamptothecins).

Derivatization can influence the properties of CPT to make the molecule more hydrophilic or more lipophilic, or that the lactone-carboxylate equilibrium is affected. In the context of the application of CPT as an anti-cancer drug, derivatization is intended to maintain or to increase activity.

"Cancer" refers to the more common forms of cancers such as bladder cancer, breast cancer, colorectal cancer, endometrial cancer, head and neck cancer, leukaemia, lung cancer, lymphoma, melanoma, non-small-cell lung cancer, ovarian cancer, prostate cancer and to childhood cancers such as brain stem glioma, cerebellar astrocytoma, cerebral astrocytoma, ependymoma, Ewing's sarcoma/family of tumors, germ cell tumor, extracranial, hodgkin's disease, leukemia, acute lymphoblastic, leukemia, acute myeloid, liver cancer, medulloblastoma, neuroblastoma, non-hodgkin's lymphoma, osteosarcoma/malignant fibrous histiocytoma of bone, retinoblastoma, rhabdomyosarcoma, soft tissue sarcoma, supratentorial primitive neuroectodermal and pineal tumors, unusual childhood cancers, visual pathway and hypothalamic glioma, Wilms' Tumor and other childhood kidney tumors and to less common cancers including acute lymphocytic leukaemia, adult acute myeloid leukaemia, adult non-hodgkin's lymphoma, brain tumor, cervical cancer, childhood cancers, childhood sarcoma, chronic lymphocytic leukaemia, chronic myeloid leukaemia, esophageal cancer, hairy cell leukaemia, kidney cancer, liver cancer, multiple myeloma, neuroblastoma, oral cancer, pancreatic cancer, primary central nervous system lymphoma, skin cancer, small-cell lung cancer.

"Carrier" refers to a diluent, adjuvant, excipient, or vehicle which is suitable for administering a diagnostic or therapeutic agent. The term also refers to a pharmaceutically acceptable component(s) that contains, complexes or is otherwise associated with an agent to facilitate the transport of such an agent to its intended target site. Carriers include those known in the art, such as liposomes, polymers, lipid complexes, serum albumin, antibodies, cyclodextrins and dextrans, chelates, or other supramolecular assemblies.

"Cationic amphiphiles" as used herein refer to cationic lipids as defined.

"Cationic liposome" refers to a liposome which has a positive net charge. The cationic liposomes are prepared from the cationic lipids or amphiphiles themselves or in admixture with other amphiphiles, particularly neutral or anionic lipids.

"Colloidal nanoaggregates" or "colloidal nanoparticle" refers to a dispersion of particles in an aqueous phase. The particles are in the size range if nanometers to micrometers i.e., they are larger that individual molecules but are not macroscopic.

"Derivative" refers to a compound derived from some other compound while maintaining its general structural features. Derivatives may be obtained for example by chemical functionalization or derivatization.

"Drug" as used herein refers to a pharmaceutically acceptable pharmacologically active substance, physiologically active substances and/or substances for diagnosis use.

"Empty" nanpoarticles or liposomes means, that the particles do not comprise the drug or active compond. In this context, "empty" is used synonymously with "drug-free".

"Encapsulation efficiency'' refers to the fraction of a compound which is encapsulated into the liposomes of a liposome suspension by a given method.

"Homogenization" refers to a physical process that achieves a uniform distribution between several components or phases. One example is high-pressure homogenisation.

"Lipid" in its conventional sense refers to a generic term encompassing fats, lipids, alcohol-ether-soluble constituents of protoplasm, which are insoluble in water. Lipids are amphiphilic molecules such as fatty acids, steroids, sterols, phospholipids, glycolipids, sulpholipids, aminolipids, or chromolipids. The term encompasses both naturally occurring and synthetic lipids. In a more general sense, lipids are characterized as amphiphiles, i.e., they are molecules which consist of lipophilic as well as hydrophilic moieties. Preferred lipids in connection with the present invention comprise at least two alkyl chains with at least 12 carbon chains and are: steroids and sterol, particularly cholesterol, phospholipids, including phosphatidyl and phosphatidylcholines and phosphatidylethanolamines, and sphingomyelins. Fatty acids could be about 12-24 carbon chains in length, containing up to 6 double bonds, and linked to the backbone. The hydrocarbon chains can be different (asymmetric), or there may be only 1 fatty acid chain present, e.g., lysolecithins. Also more than two and branced hodrocarbon chains of different chain length and structure may be present.

"Liposome" refers to a microscopic spherical membrane-enclosed vesicle (about 50-2000 nm diameter) made artificially in the laboratory. The term "liposome" encompasses any compartment enclosed by a lipid bilayer. Liposomes are also referred to as lipid vesicles.

"Lysolipid" refers to a lipid where one fatty acid ester has been cleaved resulting in a glycerol backbone bearing one free hydroxyl group.

"Lysophospholipid" refers to a phospholipid where one fatty acid ester has been cleaved resulting in a glycerol backbone bearing one free hydroxyl group.

"Negatively charged lipids" refer to lipids that have a negative net charge. Examples are phosphatidic acids, phosphatidylserines, phosphatidylglycerols, phosphatidylinositoles (not limited to a specific sugar), fatty acids, sterols.

"Neutral lipids" refer to lipids that have a neutral net charge such as cholesterol, 1,2-diacyl-glycero-3-phosphoethanolamines, 1,2-diacyl-glycero-3-phosphocholines, Sphingomyelins.

"Particle diameter" refers to the size of a particle. To experimentally determine particle diameters, dynamic light scattering (DLS) measurements, using Malvern Zetasizer 1000 or 3000 (Malvern, Herrenberg, Germany) were performed. For quantitative data analysis the average size (Z_{average}) and and the 'Polydispersity Index' (PI value), which is a measure for the accuracy of the fit and the deviation from the means size, were determined.

"Pegylated lipid" refers to a lipid bearing one ore more polyethylene glycol residues.

"Pharmaceutical composition" refers to a combination of two or more different materials with superior pharmaceutical properties than are possessed by either component.

"Phospholipid" refers to a lipid consisting of a glycerol backbone, a phosphate group and one or more fatty acids wich are bound to the glycerol backbone by ester bonds.

"Positively charged Lipids" refer to a synonym for cationic lipids (for definition see definition of "cationic lipids").

"Stabilizing agent" as used herein refers to a compound which is favourable for the stability of the inventive preparation. This might be a cryoprotectant (such as an alcohol or sugar) or an antioxidant (such as tocopherol or vitamin C).

"Sterol" refers to a steroid alcohol. Steroids are derived from the compound called cyclopentanoperhydrophenanthrene. Well-known examples of sterols include cholesterol, lanosterol, and phytosterol.

"Virtually free" of a species refers to as not detectable by HPTLC. "Virtually free of liposomes" refers to a state, where the signal from a given method such as light scattering, which is proportional to the liposome concentration, is less than 5% of the value as it is obtained in a system which has the same molecular composition but consisting of liposomes.

The inventive methods has several advantages compared with other methods known in the art. It is quick and simple and does not require complicated steps such as active or other passive loading thechniques. This has great advantages for fabrication, storage and clinical application of nanoparticulate preparations.

Fabrication is facilitated and it may be done at lower cost, since less complex components need to be produced. Many combinations of drug and nanoparticles, which have a favourable pharmacological activity, are only stable for a few hours, days, or weeks and the requirements for stability of one of the components are contrary to those of another one, for example with respect to the pH conditions, or that one of the components directly induced degradation of another one. With the so far known procedures, such preparations cannot be provided for pharmaceutical applications because the shelf life is too short. With the enclosed procedure, even preparations, which have a stability of only few hours, might be provided for regular pharmaceutical application.

Large-scale production and sufficient shelf life of loaded cationic nanoparticles, particularly liposomes, are a paramount problem in many cases, which might inhibit the development of a pharmaceutical application. Active agent or drug free nanoparticles however can be stored for long a time, in liquid form or as lyophilisates, even in cases, where the lipid composition is complex. Therefore, nanoparticles with an optimised composition/formulation for better pharmacological efficacy become available for regular application as carrier. For example, with polymer graft nanoparticles such as liposomes, which can be used to reduce serum interactions, lyophilization is difficult. Further, in drug-loaded liquid formulations the drug may be released from the nanoparticles during storage, but the drug-free, empty liposomes may be stored much longer. Thus, in the latter case the present invention is suitable and provides a useful method for producing a pharmaceutically active preparation.

A further advantage is that production may be less complex and expensive. For example, often it is necessary to lyophilise liposome formulations in order to provide sufficient shelf life. In many cases the reason is, that the active agent is released from the liposomes, wherein nanoparticles without agent would be stable for much longer time. In such a case, the novel method makes a lyophilization step redundant and since active compound and nanoparticles can be stored separately.

With the enclosed technique, a preparation with favourable properties, which are different to those of the individual components, is obtained. The preparation which is obtained by loading cationic nanoparticles, particularly liposomes, with an active agent such as camptothecin carboxylate, has a better pharmacological activity compared with the individual components. This is especially true for camptothecin carboxylate which is known to cause severe side effects in patients.

Another advantage of adding the drug only immediately before use to the liposomes is, that dosing of the drug and the lipid fraction (nanoparticles) can be adjusted independently according to the needs of an individual patient.

Summarizing, the inventive method has the following advantages:
- It is a quick and easy technique for loading cationic nanoparticles.
- Compositions produced by use of the inventive method provide preparations with improved pharmacological activity with respect to the individual components (see step a) and b)).
- Production and storage of the two components (step a) and b)) separately is easier and less complex and thereby less expensive.
- Production and storage conditions can be optimised for the individual components.
- Formulations for components which would induce degradation of one another during storage can be realized.
- Favourable lipid and drug combinations can be realized, which would otherwise not be possible.
- Better dosing can be achieved, since lipid and drug content can be selected independently.

The cationic colloidal nanoparticles of the present invention may comprise as cationic constituent amphiphiles, polymers, particularly polyelectrolytes, or other.

The inventive preparation preferably comprises cationic amphiphiles, which are selected from lipids, lysolipids or pegylated lipids having a positive net charge. The lipid may comprise one or more hydrocarbon chains, which are not necessarily identical, which are branched or unbranched, saturated or unsaturated with a mean chain length from C12 to C24.

Useful cationic lipids for the present invention include:

DDAB, dimethyldioctadecyl ammonium bromide; N-[1-(2,3-dioleoyloxy)propyl]-N,N,N-trimethyl ammonium (DOTAP); N-[1-(2,3-diacyloxy)propyl]-N,N,N-trimethyl ammonium, (including but not limited to: dioleoyl, dimyristoyl, dilauroyl, dipalmitoyl and distearoyl; also two different acyl chains can be linked to the glycerol backbone); N-[1-(2,3-dioloyloxy)propyl]-N,N-dimethyl amine (DODAP); N-[1-(2,3-diacyloxy)propyl]-N,N-dimethyl amine, (including but not limited to: dioleoyl, dimyristoyl, dilauroyl, dipalmitoyl and distearoyl; also two different acyl chains can be linked to the glycerol backbone); N-[1-(2,3-dioleyloxy)propyl]-N,N,N-trimethylammonium (DOTMA); N-[1-(2,3-dialkyloxy)propyl]-N,N,N-trimethyl ammonium, (including but not limited to: dioleyl, dimyristyl, dilauryl, dipalmityl and distearyl; also two different alkyl chains can be linked to the glycerol backbone); dioctadecylamidoglycylspermine (DOGS);, 3□-[N-(N',N'-dimethylaminoethane)carbamoyl]cholesterol (DC-Chol); 2,3-dioleoyloxy-N-(2-(sperminecarboxamido)-ethyl)-N,N-dimethyl-1-propanaminium trifluoro-acetate (DOSPA); β-alanyl cholesterol; cetyl trimethyl ammonium bromide (CTAB); diC14-amidine; N-*tert*-butyl-N'-tetradecyl-3-tetradecylaminopropionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl(alkenyl)-3-(2-hydroxyethyl)-imidazolinium chloride derivatives as described by Solodin et al. (1995) Biochem. 43:13537-13544, such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, contain a hydroxyalkyl moiety on the quaternary amine, as described e.g. by Felgner et al. [Felgner et al. *J. Biol. Chem.* 1994, 269, 2550-2561] such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. [US5498633].
In a preferred embodiment the cationic lipid is selected from a quaternary ammonium salt such as N-[1-(2,3-diacyloxy)propyl]-N,N,N-trimethyl ammonium, wherein a pharmaceutically acceptable counter anion of the quaternary amino compound is selected from the group consisting of chloride, bromide, fluoride, iodide, nitrate, sulfate, methyl suifate, phosphate, acetate, benzoate, citrate, glutamate or lactate. In a more preferred embodiment, the the cationic lipid is DOTAP.

The inventive preparation can further comprise amphiphiles with a negative and/or neutral net charge (anionic and/or neutral amphiphile). These can be selected from sterols or lipids such as cholesterol, phospholipids, lysolipids, lysophospholipids, sphingolipids or pegylated lipids with a negative or neutral net change. Useful anionic and neutral lipids thereby include: Phosphatidic acid, phosphatidylserine, phosphatidylglycerol, phosphatidylinositol (not limited to a specific sugar), fatty acids, sterols containing a carboxylic acid group, cholesterol, 1,2-diacyl-sn-glycero-3-phosphoethanolamine, including but not limited to dioleoyl (DOPE), 1,2-diacyl-glycero-3-phosphocholines, sphingomyelin. The fatty acids linked to the glycerol backbone are not limited to a specific length or number of double bonds. Phospholipids may also have two different fatty acids. Preferably the further lipids are in the liquid crystalline state at room temperature and they are miscible (i.e. a uniform phase can be formed and no phase separation or domain formation occurs) with the used cationic amphiphile, in the ratio as they are applied.

In a preferred embodiment the neutral amphiphile is a phosphatidylcholine.

In a further preferred embodiment the inventive preparation may comprise at least one further amphiphile in an amount of about 0 to about 70 mol%, preferably of about 20 mol% to about 50 mol% and most preferably of about 30 mol% to about 40 mol% based on the total amphiphile concentration.

The present invention may further comprise a stabilizing agent, which is selected from a sugar or a polyvalent alcohol or a combination thereof such as trehalose, maltose, sucrose, glucose, lactose, dextran, mannitol or sorbitol. In a preferred embodiment the stabilizing agent is trehalose or glucose.

In a preferred embodiment the inventive preparation comprises a camptothecin drug in its carboxylate form in the range of about 0.1 mol % to less than about 100 mol% with respect to the amount of cationic lipid. In other embodiments it is present from about 1 mol% to about 50 mol%. In other embodiments, a camptothecin drug is present in about 3 mol% to about 30 mol% and in even other embodiments it is present in about 5 mol% to about 10 mol%. The content of CPT in its lactone form is thereby below about 10% (% means molar fraction of the total CPT content), preferably below about 8% and more preferably below about 6% and most preferably below about 4% with respect to total CPT.

It is a further object of the present invention to provide a kit comprising a) a camptothecin drug in the carboxylate form, b) drug free cationic nanoparticles and optionally c) an aqueous medium, wherein the components a), b) and optionally c) are in separate containers. Nanoparticles, as well as active agent are thereby stored individually and mixed together directly before use, optionally in a suitable aqueous solution such as water or buffer.

The kit is thereby suitable for the manufacture of a pharmaceutical compostion. Accordingly, the present invention provides a pharmaceutical composition comprising the inventive preparation, optionally together with a pharmaceutically acceptable carrier, diluent and/or adjuvant.

The inventive kit, as well as the pharmaceutical composition is suitable for the manufacture of a medicament to treat an angiogenesis associated disease such as cancer.

An angiogenesis associated disease is dependent on blood supply. The local interruption of the vasculature will produce an avalanche of cell death. The vascular endothelium is in direct contact with the blood. It is contemplated that a variety of diseases can be prevented and treated with the foregoing methods and compositions. In a preferred embodiment, a medicament manufactured by using the present invention may be useful for preventing and/or treating a disease such as cancer, a variety of inflammatory diseases, diabetic retinopathy, rheumatoid arthritis, inflammation, dermatitis, psoriasis, stomach ulcers, macular degeneration, hematogenous and solid tumors. In a further preferred embodiment, it can be applied for producing a medicament for preventing and/or treating solid tumors and their metastases such as bladder, brain, breast, cervical, colorectal, endometrial, head and neck or kidney cancer, leukemia, liver or lung cancer, lymphoma, melanoma, non-small-cell lung; ovarian, pancreatic or prostate cancer.

### Examples

The following examples illustrate the invention and are non-limiting embodiments of the invention claimed below.

### I. Comparison of normally produced liposomes liposomes produced by the inventive method

In order to demonstrate the general characteristics of the preparations produced by the enclosed approach, and to compare them with liposomes, which have been loaded with a drug or a compound in the standard way a selection of examples for the loading of cationic liposomes with camptothecin carboxylate and with aminofluorescein by the enclosed method is given. For simplicity and better comparison between the different experimental conditions, all examples are made with DOTAP or DOTAP/DOPC mixtures.

For direct comparison, CPT-carboxylate loaded liposomes were made by one of the techniques as given in the literature. Liposomes, loaded with CPT-carboxylate by the enclosed procedure were prepared with the same total composition. The fraction of free (non-liposomally bound) CPT was determined in both cases. The free CPT was determined by centrifugation with the centrifugal concentrator Vivaspin 2 (Vivasciences). By a membrane of MWCO 100 kDa, the molecularly dissolved solutes were separated from the colloidal particles. The concentration of CPT in the filtrate was determined by UV-spectroscopy. All measurements with camptothecin were made with tris/HCl buffer, pH 7.5,10 mM or 20 mM.

For the production of the classically made formulations, the liposomes were formed directly in the aqueous solution of CPT carboxylate. By subsequent extrusion through membranes of 200 nm pore size homogeneous mixing and encapsulation was provided. The liposomes were made either by the 'film method' or by 'ethanol injection'.

For the film method, a solution of the lipids in chloroform is evaporated in a round bottom flask. A thin dry lipid film is formed at the inner wall of the flask. For the production of empty liposomes, the film is reconstituted with water or buffer solution. For the production of CPT loaded liposomes, the film is reconstituted with the CPT-carboxylate solution. In both cases the so-formed multilamellar vesicle suspension is extruded through membranes of 200 nm pore size in order to obtain monodisperse, monolamellar liposomes.

For the ethanol injection, a concentrated solution of the lipid in ethanol (typically 400 mM) is injected into the aqueous phase. Extrusion is perfomed is the same way with the film method. Formulations which have been produced by ethanol injection subsequently have been lyophilized for storage. For lyophilization, standard protocols were applied. By the lyophilization, in addition to the water, also the ethanol was removed from the preparations. Before use the lyophilisates were reconstituted with water.

### Example 1

### Liquid formulations at different CPT and DOTAP concentrations

Formulations were prepared by the classical standard procedure (film method) as reference liposomes and with the enclosed procedure at different CPT and DOTAP concentrations, Tris/HCl pH 7.5, 20 mM. The results are given in Table 2 and 3.

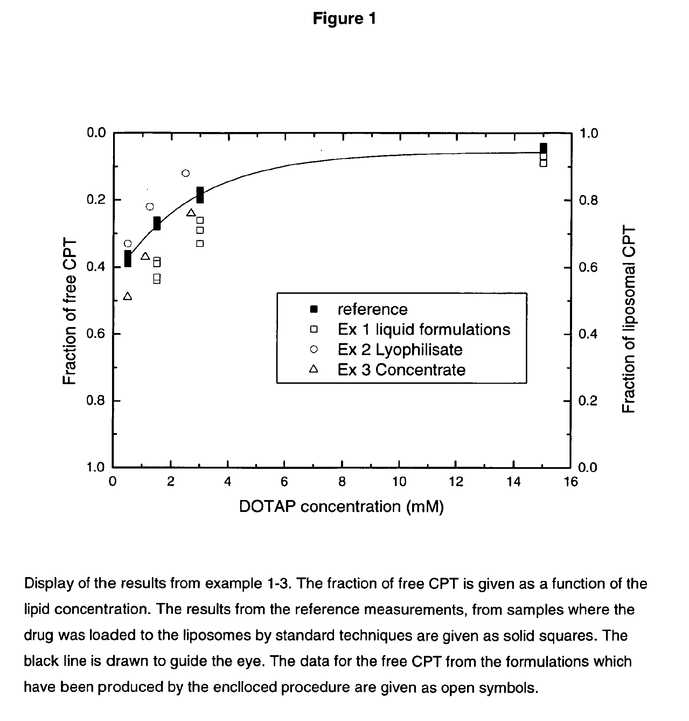

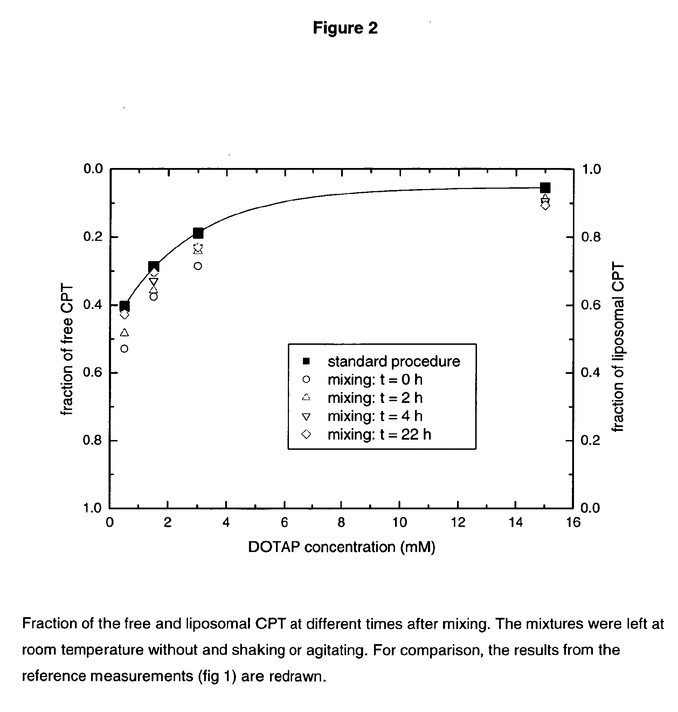

As can be seen from the results, in the complete range of tested concentrations, with the enclosed method, the liposome-bound fraction of CPT is very similar to that of normally produced CPT loaded liposomes (reference). For better comparison the results are graphically displayed in Figure 1 as a function of the lipid concentration.

### Example 2

### Reconstitution of DOTAP lyophilisates with CPT carboxylate solution

A lyophilisate of DOTAP liposomes (30 mM) was reconstituted with 2.088 ml of an aqueous solution of CPT carboxylate in water (1.4 mM). Subsequently 0.252 ml of 100 mM Tris/HCl buffer, pH 7.5 were added. The original volume of the pure DOTAP liposome suspension was 2.1 ml. The final concentration of DOTAP was 27 mM and the CPT concentration was 1.25 mM. From the resulting preparation the fraction of free CPT was determined by centrifugation at different lipid concentrations.
The results show an analogous behaviour as in the previous section: most of the CPT is bound to the liposomes, and the fraction of free CPT is higher for lower lipid concentration. This demonstrates, that lyophilisates of liposomes can be reconstituted directly with a CPT solution and CPT loaded liposome suspensions are achieved (Fig. 1). Results are given in Table 4.

### Example 3

### Concentrated DOTAP preparations in water + CPT carboxylate

A concentrated preparation of DOTAP in water was prepared by high pressure homogenization. The concentration of the preparation was about 270 mM. 1 ml of the DOTAP concentrate and 9 ml of CPT carboxylate solution, c= 1.55 M were mixed. The resulting DOTAP concentration was 27 mM and the CPT concentration was 1.4 mM, pH 7.5. This preparation was diluted 1:10 1:25 and 1.50 and the fraction of free CPT was determined. The results are given in Table 5 and are displayed in Figure 1.

In Fig. 1 the results for the free CPT from Examples 1-3 are displayed as a function of lipid concentration (open symbols). For comparison, the data for classically prepared DOTAP/CPT liposomes are given (solid squares). The solid line is drawn to guide the eye. As can be seen, all results from Examples 1-3 are in the same range as the reference. The deviations are in the range of the accuracy of the method. They may may be due to small differences in the environmental conditions between the individual measurements.

### Example 4

### Time scale for the formation of the CPT/DOTAP complex

In this example the kinetics of self-loading of DOTAP-liposomes with CPT carboxylate was investigated.
Liquid DOTAP formulations were exposed to CPT-carboxylate solutions for different time scales. The DOTAP concentration was 15 mM and the CPT carboxylate concentration was 0.75 mM. After the given time, the fraction of free CPT was determined for different dilutions. The time scale for one measurement (given by the necessary centrifugation time) is in the order of about 40 min. As for the previous experiments, the measurements were performed in Tris/HCl, pH 7.5: The buffer concentration was 10 mM. The results are summarized in Fig. 2. For comparison, the data from a preparation as produced by the standard film method as a reference which is displayed also in Fig. 1 are given.

As can be seen, in accordance with the results from Fig. 1, already directly after mixing the fraction of free CPT is very similar to that of the classical formulation. At the original concentration of the formulation, 15 mM, already for the first time point, loading reached saturation, i.e., no further changes were noted for the subsequent measurements. For the diluted samples an increase of the fraction of loaded drug was observed up to 4 hours. Because the samples were not stirred during the exposition period, in the diluted measurements diffusion limited transport of the camptothecin to the liposome may have played a role for the somewhat slower loading.

### Example 5

### Loading of camptothecin carboxylate into DOTAP/DOPC mixtures

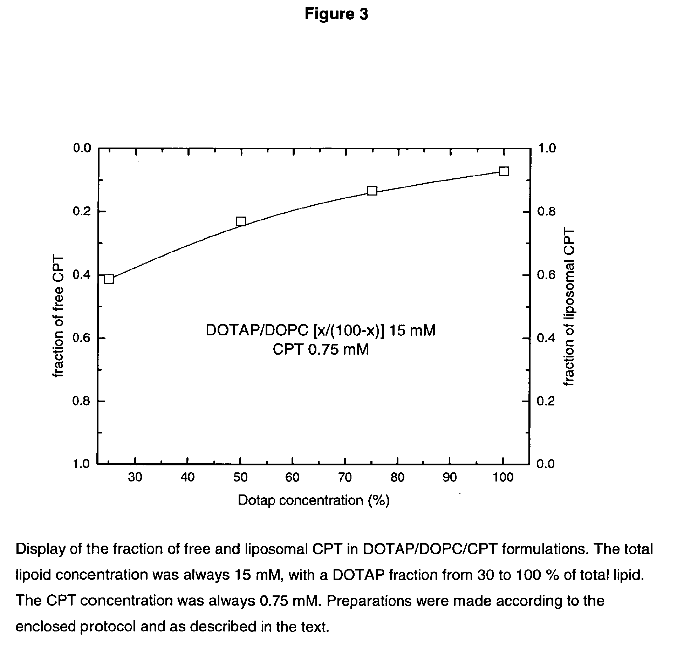

The enclosed procedure was applied for the loading of DOTAP/DOPC liposomes with camptothecin carboxylate. In Fig. 3 the results for the loading of DOPTA/DOPC liposomes with different molar fractions (30-100% DOTAP) of are shown. The procedure for the loading and determination of the free camptothecin were analogous to those of Examples 1-4. As ca be seen, also for lipid mixtures which comprise non-cationic liposomes efficient loading is possible. By the additional presence of DOPC in the liposme, the loading efficacy is only slightly reduced.

### Example 6

### Loading of aminofluorescein to DOTAP/DOPC liposomes

A 25 mM liposome formulation consisting of DOTAP/DOPC 1:1 in a solution of 5% glucose (w/v) was prepared. Briefly, a solution of DOTAP/DOPC in chloroform was put into a round bottom flask, and the solvent was evaporated in order to obtain a thin lipid film. The lipid film was reconstituted with the glucose solution to a total lipid concentration of 25 mM. The resulting multilamellar, polydisperse liposome suspension was extruded through a membrane of 200 nm pore size to obtain liposomes of uniform size.
10 ml of the liposome preparation and 10 ml of a 5 mM aqueous solution of aminofluorescein were combined.
Subsequently, the molecularly dissolved components were removed by cross-flow filtration, using a VIVAFLOW filtration kit, MWCO=50,0000 and a Masterflex easy lod pump, model. Procedure:
20 ml of the preparation were diluted with 20 ml glucose. By the subsequent dialysis, part of the solvent and the molecularly dissolved (low molecular) compound penetrated across the separation membrane, while the liposomes were retained. Fiitration was performed until the volume of the original solution was reduced to half of the start volume. Then the lost volume was substituted by glucose solution and the filtration was started again. Three filtration cycles were performed.
The concentration of aminofluorescein in the permeate and in the retained liposome suspension was determined by UV-vis spectroscopy.
The concentration of aminofluorescein in the filtrate decreased rapidly to a very low equilibrium value. By the eye only a faint yellowish colour could be made out. The amount of aminofluorescein which was retained with the liposome suspension after three cycles of filtration was 47 % of the original concentration.

The results demonstrate, that also compound other than camptothecin carboxylate can be loaded by the enclosed procedure to cationic liposomes. The retained amount was four times as high as in case of the expected value without any retention. Because the experimental setup and the conditions were different to the previously described experiments, a quantitative comparison between the retention efficacy for the two compounds is not possible.

### Example 7

### Solubility of camptothecin in an aqueous phase at different pH values

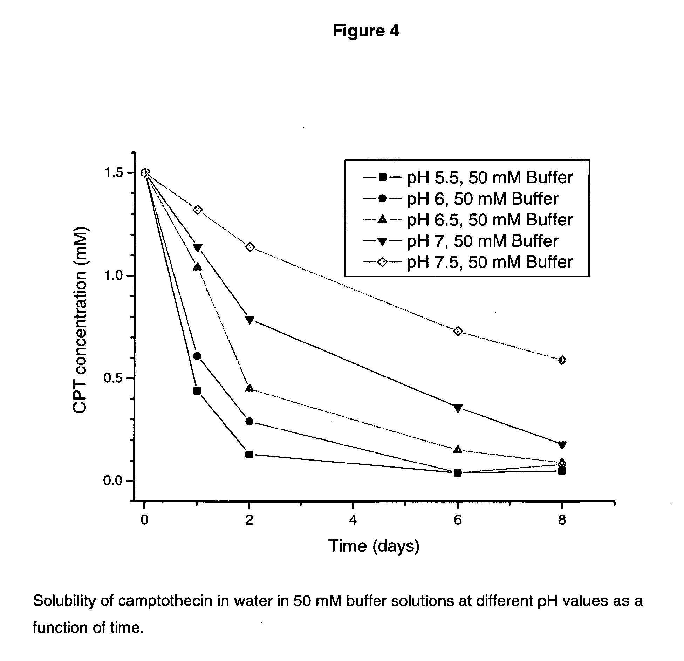

In this measurements, the solubility of camptothecin in aqueous media at different pH values is investigated. Pure camptothecin carboxylate was dissolved in a buffered (50 mM) aqueous phase at different pH values. At different times the solutions were centrifuged in order to remove camptothecin lactone crystals and the remaining concentration of the camptothecin in the supernatant was determined by UV-vis spectroscopy. The data are given in Fig. 4. As can be seen, at pH values below 7, the concentration of the camptothecin reaches very low values within few days. These concentrations are too low for sufficient paharmaceiútical efficacy.

### Example 8

### Stability of camptothecin carboxylat in concentrated alkaline media

Camtothecin in the lactone form was dissolved at a concentration of 4.6 µg/ml in concentrated ammonia (NH₄OH in order to obtain the carboxylate. HPLC analysis was performed directly after dissolving the lactone and after 19 days. In the HPLC chromatograms, no indication for a degradation of the camptothecin by the alkaline medium.

### Examples 9 and 10

### Preparation A

In this example an empty liposomal preparation (liposomes not loaded with the drug) was prepared by applying high-pressure homogenization.

### Preparation of the empty liposomal preparation

### Raw dispersion:

2.34 g DOTAP-Cl were weighted in a 500 ml round bottom flask. 225 ml trehalose (9%, m/m) were added to a final DOTAP content of 15 mM. The inhomogeneous mixture was intensively stirred for 25 minutes to form a more homogeneous liposomal raw dispersion.

### High-pressure homogenization:

This raw dispersion was homogenized using a high-pressure homogenization device from Avestin (Emulsiflex C5, Canada). During homogenizing the liposomal preparation was cooled at 4°C. After two homogenizing runs with a pressure of 500bar a very homogeneous opalescent liposomal dispersion was obtained. The homogenizing steps were performed without any problems with a constant flow.

### Extrusion:

One extrusion step was performed through a polycarbonate membrane filter unit (Osmonics, 220 nm pore size) without any problems.

### Liposomal size and size distribution:

The sample was diluted 1:10 with trehalose (9%) and was measured by dynamic light scattering (Malvern device). Preparations had a Z_{Ave} [nm] before extrusion of about 150 nm to about 130 nm and after extrusion of about 120 nm. PI values were all about 0.5.

### HPLC Analysis:

HPLC Analysis was used to measure concentration and impurities of DOTAP of liposomal preparation. The latter were all in the range of about 2.6 area%.

### pH Analysis:

The final liposomal preparation had a pH value of 5.6. Prior to measurement the liposomal preparation was dilution with an aqueous NaCl solution (20mM).

### Preparation of aqueous CPT-Na solution

Camptothecin in its lactone form was suspended in an aqueous NaOH solution. The molar ratio of CPT/NaOH was 1:1.05. The final CPT concentration was 0.75mM. The inhomogeneous mixture (CPT lactone is not water-soluble) was warm to 50°C. After 2 hours of intensive stirring a clear solution of sodium carboxylate was obtained. The solution was filtered through a 0.45µm PVDF membrane filter to removed possible particles of remaining non-reacted CPT lactone. The final solution typically has a pH of 11.2.

Part of the basic CPT solution was used to adjust the pH at 7.4 by adding 240µl HCl (0.1 M) to 10ml of the empty liposomes.

### Analysis of the CPT-Na solution

### HPLC Analysis:

The total CPT concentration of both CPT solutions (pH 11.2 & 7.4) was determined as 0.75mM. The CPT lactone content was less than 1% (molar fraction of the total CPT content) in both solutions.

### Mixing the liposomal preparation with the aqueous CPT solution

Two different liposomal CPT preparations were prepared:
- TM213: empty liposomes with the aqueous CPT solution, pH 7.4
- TM214: empty liposomes with the aqueous CPT solution, pH 11.2

### Procedure:

2.4ml of the respective aqueous CPT solution was added to stirred empty liposomes. After 10 minutes stirring both liposomal CPT preparations were into vials and were freeze-dried.

The pH of the resulting liposomal preparation of both mixtures (TM213 and TM214) had a pH between 6.3 and 7.0.

### Analysis of the liposomal CPT preparation after reconstitution of the lyophilisates

The lyophilisates were reconstitution with water. The amount of water was calculated to reached the concentration of the preparation prior freeze-drying. After 30 min storing the freshly reconstituted preparation analysis was performed.

### Liposomal size and size distribution:

The sample was diluted 1:10 with trehalose (9%) and was measured by dynamic light scattering (Malvern device):

### Liposomal size and size distribution after each step

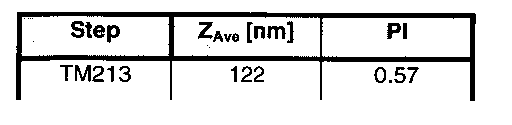

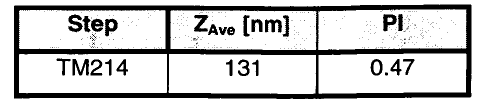

### HPLC Analysis:

HPLC Analysis was used to measure concentration and impurities of DOTAP of liposomal preparation.

### HPLC results

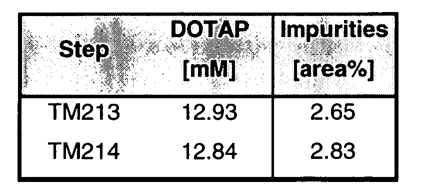

### pH Analysis:

The pH of both formulations were in the same range: 5.9 (TM213) and 5.5 (TM214)

### Preparation B

In this example an empty liposomal preparation (liposomes not loaded with the drug) was prepared by ethanol injection.

### Preparation of the empty liposomal preparation

2.34 g DOTAP-Cl were dissolved in ethanol reaching a DOTAP concentration of 400mM. The ethanolic solution was injected rapidly into an aqueous trehalose solution (9%, mass/mass) to a final DOTAP content of 15 mM. The formed raw dispersion was extruded three times through a polycarbonate membrane filter unit (Osmonics, 220 nm pore size) without any problems.

### Analysis of the final empty liposomal preparation:

### Liposomal size and size distribution:

The sample was diluted 1:10 with trehalose (9%) and was measured by dynamic light scattering (Malvern device):
Zave: 175 nm, PI: 0.20

### HPLC Analysis:

DOTAP: 14mM
Impurities: 2.1 area%

### pH Analysis:

The final liposomal preparation had a pH value of 5.6. Prior to measurement the liposomal preparation was dilution with an aqueous NaCl solution (20mM).

### Preparation of aqueous CPT-Na solution

Analogously to the procedure described above.

### Mixing the liposomal preparation with the aqueous CPT solution

Two different liposomal CPT preparations were prepared:
- TM213: empty liposomes with the aqueous CPT solution, pH 7.4
- TM214: empty liposomes with the aqueous CPT solution, pH 11.2

### Procedure:

2.4ml of the respective aqueous CPT solution was added to stirred empty liposomes. After 10 minutes stirring both liposomal CPT preparations were into vials and were freeze-dried.

Result: Freeze-drying was performed without any problems.

The pH of the resulting liposomal preparation of both mixtures (TM213 and TM214) had a pH between 6.3 and 7.0.

### Analysis of the liposomal CPT preparation

The lyophilisates were reconstitution with water. The amount of water was calculated to reached the concentration of the preparation prior freeze-drying. After 30 min storing the freshly reconstituted preparation analysis was performed.

### Liposomal size and size distribution:

The sample was diluted 1:10 with trehalose (9%) and was measured by dynamic light scattering (Malvern device):

### Liposomal size and size distribution after each step

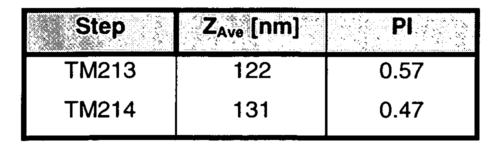

### HPLC Analysis:

HPLC Analysis was used to measure concentration and impurities of DOTAP of liposomal preparation.

### HPLC results

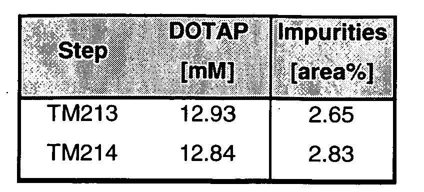

### pH Analysis:

The pH of both formulations were in the same range: 5.9 (TM213) and 5.5 (TM214)

Result: Data show excellent analytic results and proof of concept

### Stability

### Empty liposomal preparation

Liquid empty liposomal preparation, manufactured by either high-pressure homogenization or ethanol injection, were stored at 4°C. According the crucial analysis of liposomal size, size distribution, DOTAP content and DOTAP impurity a stability of at least 3 months has been observed. It was observed that single preparations had a stability of at least one year.

If liquid empty liposomal preparation has been freeze-dried, stability (storage at 4°C) of at least 6 months has been observed. It was observed that single preparations had a stability of at least one and a half year.

### Aqueous CPT-Na solution

The aqueous CPT-Na solution prepared as described before were tested on storage stability at 4°C. After storing one month no change of CPT content, CPT impurity or pH has been observed. Also the content of CPT lactone did not change. Preliminary results from a stability study at 25°C (accelerated stability study) indicate stability at 4°C of at least 3 month.

### Stability of the final liposomal CPT preparation (lyophilisates)

A liposomal CPT preparation has been manufactured by mixing empty liposomes (high-pressure homogenization) and a aqueous CPT-Na (pH 11 and pH adjusted at 7.4) followed by freeze-drying.

### In-use stability at 25°C after reconstitution:

No significant change of critical formulation-related parameters has been observed within 4h during storing at 25°C after reconstitution.

### Temperature-stress study of final liposomal CPT lyophilisates:

Lyophilisates (as described above) were stored at 50°C for 3 days. No DOTAP degradation could be observed.

### Example 11

### Human Therapy Treatment Protocols

This example is concerned with human treatment protocols using the preparations and suspensions disclosed. Treatment will be of use for diagnosing and/or treating various human conditions and disorders associated with enhanced angiogenic activity. It is considered to be particularly useful in anti-tumor therapy, for example, in treating patients with solid tumors and hematological malignancies or in therapy against a variety of chronic inflammatory diseases such as psoriasis.

A feature of the invention is that several classes of diseases and/or abnormalities are treated without directly treating the tissue involved in the abnormality e.g., by inhibiting angiogenesis the blood supply to a tumor is cut off and the tumor is killed without directly treating the tumor cells in any manner.

Methods of treating such patients using lipid:drug complexes have already been formulated. It is contemplated that such methods may be straightforwardly adapted for use with the method described herein. As discussed above, other therapeutic agents could be administered either simultaneously or at distinct times. One may therefore employ either a pre-mixed pharmacological composition or "cocktail" of the therapeutic agents, or alternatively, employ distinct aliquots of the agents from separate containers.

The various elements of conducting a clinical trial, including patient treatment and monitoring, will be known to those of skill in the art in light of the present disclosure.

For regulatory approval purposes, it is contemplated that patients chosen for a study would have failed to respond to at least one course of conventional therapy and would have objectively measurable disease as determined by physical examination, laboratory techniques, or radiographic procedures. Such patients would also have no history of cardiac or renal disease and any chemotherapy should be stopped at least 2 weeks before entry into the study.

The required application volume is calculated from the patient's body weight and the dose schedule. Prior to application, the formulation can be reconstituted in an aqueous solution. Again, the required application volume is calculated from the patient's body weight and the dose schedule.

The disclosed formulations may be administered over a short infusion time. The infusion given at any dose level should be dependent upon the toxicity achieved after each. Hence, if Grade II toxicity was reached after any single infusion, or at a particular period of time for a steady rate infusion, further doses should be withheld or the steady rate infusion stopped unless toxicity improved. Increasing doses should be administered to groups of patients until approximately 60% of patients showed unacceptable Grade III or IV toxicity in any category. Doses that are 2/3 of this value would be defined as the safe dose.

Physical examination, tumor measurements, and laboratory tests should, of course, be performed before treatment and at intervals of about 3-4 weeks later. Laboratory tests should include complete blood counts, serum creatinine, creatine kinase, electrolytes, urea, nitrogen, SGOT, bilirubin, albumin, and total serum protein.

Clinical responses may be defined by acceptable measure or changes in laboratory values e.g. tumormarkers. For example, a complete response may be defined by the disappearance of all measurable disease for at least a month. Whereas a partial response may be defined by a 50% or greater reduction.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the composition, methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### Administration and dosing

The present invention includes a method of delivery of a pharmaceutically effective amount of the inventive preparation or liposome suspension obtainable thereof comprising an active compound to an angiogenic vascular target site of a subject in need thereof. A "subject in need thereof" thereby refers to a mammal, e. g. a human.

The route of administration comprises peritoneal, parenteral or topic administration and the formulations are easily administered in a variety of dosage forms such as implantation depots, injectable solutions and the like.

For use with the present invention the term "pharmacologically effective amount" of a compound administered to a subject in need thereof (which may be any animal with a circulatory system with endothelial cells which undergo angiogenesis) will vary depending on a wide range of factors. For example, it would be necessary to provide substantially larger doses to humans than to smaller animal. The amount of the compound will depend upon the size, age, sex, weight, and condition of the patient as well as the potency of the substance being administered. Having indicated that there is considerable variability in terms of dosing, it is believed that those skilled in the art can, using the present disclosure, readily determine appropriate dosing by first administering extremely small amounts and incrementally increasing the dose until the desired results are obtained. Although the amount of the dose will vary greatly based on factors as described above, in general, the present invention makes it possible to administer substantially smaller amounts of any substance as compared with delivery systems which target the surrounding tissue e. g., target the tumor cells themselves.

The pharmaceutically effective amount of a therapeutic agent as disclosed herein depends on the kind and the type of action of the agent. For the examples mentioned here, it is within the range of about 0.1 to about 20 mg/kg in humans.

The pharmaceutically effective amount of a diagnostic agent as disclosed herein depends on the type of diagnostic agent. The exact dose depends on the molecular weight of the compound, and on the type and the intensity of the signal to be detected. For the examples as given here (fluorescein as fluorescence dye, gadolinium complexes as MRI markers), the applied dose may range from about 0.1 to 20 mg/kg. Most frequent doses are in the order of about 5 mg/kg.

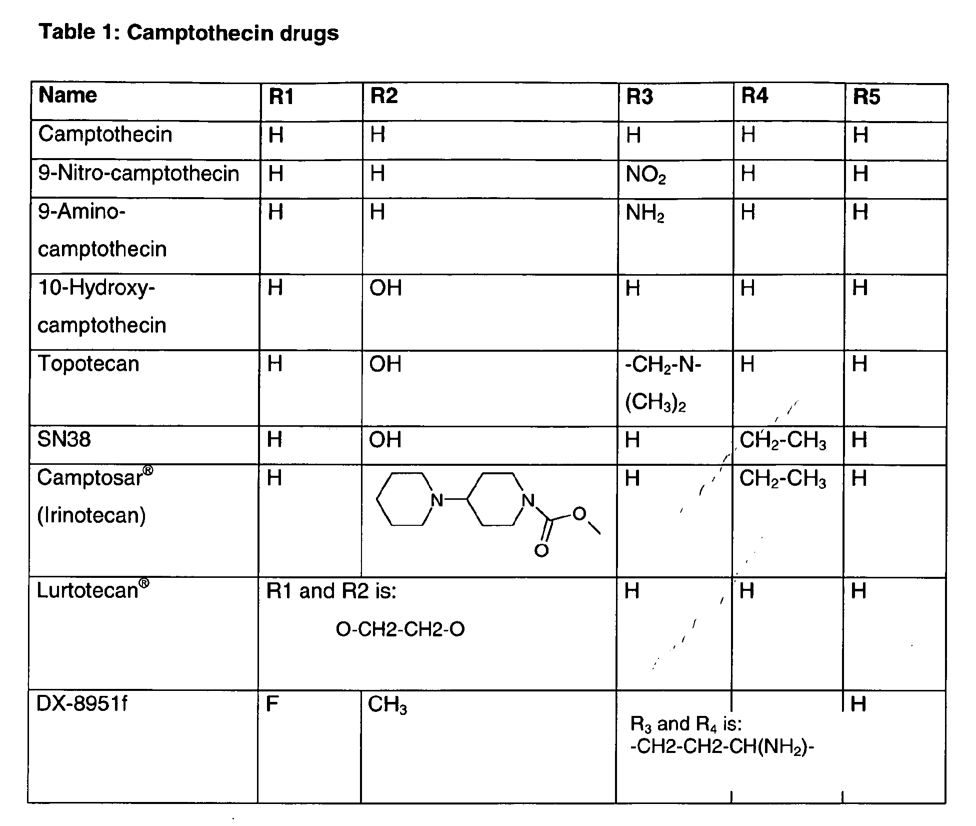

**Table 2**

| **Reference formulations (1 - 10 mol% CPT) in Tris/HCl 20 mM, , pH 7.5 (Film method)** | | | | |
|---|---|---|---|---|
| | **conc DOTAP (mM)** | **conc CPT (total) (µM)** | **conc CPT (filtrate) (µM)** | **C**_{**filtrate**}**/C**_{**total**} |
| **1** | **0.5** | **5** | **1.8** | **0.36** |
| **2** | **1.5** | **15** | **4.1** | **0.27** |
| **3** | **3** | **30** | **5.8** | **0.19** |
| **4** | **15** | **150** | **8.8** | **0.06** |
| **5** | **0.5** | **15** | **5.8** | **0.39** |
| **6** | **1.5** | **45** | **12.5** | **0.28** |
| **7** | **3** | **90** | **18.1** | **0.20** |
| **8** | **15** | **450** | **26.7** | **0.06** |
| **9** | **0.5** | **25** | **9.3** | **0.37** |
| **10** | **1.5** | **75** | **19.2** | **0.26** |
| **11** | **3** | **150** | **29.4** | **0.20** |
| **12** | **15** | **750** | **47.4** | **0.60** |
| **13** | **0.5** | **35** | **13.3** | **0.38** |
| **14** | **1.5** | **10,5** | **27.7** | **0.26** |
| **15** | **3** | **21** | **39.5** | **0.19** |
| **16** | **15** | **105** | **58.4** | **0.06** |
| **17** | **0.5** | **50** | **19.4** | **0.39** |
| **18** | **1.5** | **150** | **38.3** | **0.26** |
| **19** | **3** | **300** | **51.7** | **0.17** |
| **20** | **15** | **1500** | **66.9** | **0.04** |

**Table 3**

| **Preparations as produced by the enclosed method in Tris/HCl, 20 mM, pH 7.5. Solutions of CPT-carboxylate were exposed to DOTAP liposome suspensions at a variety of concentrations** | | | | |
|---|---|---|---|---|
| | **conc DOTAP (mM)** | **conc CPT (total) (µM)** | **conc CPT(filtrate) (µM)** | **C**_{**fitrate**}**/C**_{**total**} |
| **1** | **0.5** | **4** | **2.3** | **0.58** |
| **2** | **1.5** | **12** | **5.1** | **0.43** |
| **3** | **3** | **24** | **6.9** | **0.29** |
| **4** | **15** | **120** | **8.5** | **0.07** |
| **5** | **0.5** | **10.2** | **5.6** | **0.54** |
| **6** | **1.5** | **30.8** | **12.2** | **0.39** |
| **7** | **3** | **61.6** | **15.9** | **0.26** |
| **8** | **15** | **308** | **22.8** | **0.07** |
| **9** | **0.5** | **19.9** | **13.2** | **0.66** |
| **10** | **1.5** | **59.7** | **25.5** | **0.43** |
| **11** | **3** | **119.4** | **35.1** | **0.29** |
| **12** | **15** | **597** | **54.6** | **0.09** |
| **13** | **0.5** | **28.7** | **17.8** | **0.62** |
| **14** | **1.5** | **86** | **38.2** | **0.44** |
| **15** | **3** | **172** | **56.8** | **0.33** |
| **16** | **15** | **860** | **79.9** | **0.09** |
| **17** | **0.5** | **44** | **24.2** | **0.55** |
| **18** | **1.5** | **132** | **50.7** | **0.38** |
| **19** | **3** | **264** | **70.1** | **0.26** |
| **20** | **15** | **1320** | **93.6** | **0.07** |

**Table 4**

| **Preparations as produced by reconstitution of DOTAP lyophilisates with solutions of CPT-carboxylate. Measurements of the fraction of free CPT were performed at a variety of concentrations after dilution of the original formulation. The aqueous phase contained 10mM Tris/HCl, pH 7.5 for the measurements.** | | | |
|---|---|---|---|
| | **DOTAP conc. (mM)** | **CPT total conc. (mM)** | **Fraction of free CPT C**_{**free**}**/C**_{**0**} |
| **1** | **2.5** | **0.125** | **0.12** |
| **2** | **1.25** | **0.0675** | **0.22** |
| **3** | **0.5** | **0.025** | **0.33** |

**Table 5**

| **Preparations as produced by exposing concentrated DOTAP formulations with CPT carboxylate formulations. Measurements of the fraction of free CPT were performed at different dilutions after forming the preparation. The aqueous phase contained 10mM Tris/HCl, pH 7.5 for the measurements.** | | | |
|---|---|---|---|
| | **DOTAP conc. (mM)** | **CPT total. (mM)** | **Fraction of free CPT cfree/C**_{**total**} |
| **1** | **2.7** | **0.14** | **0.24** |
| **2** | **1.1** | **0.056** | **0.37** |
| **3** | **0.5** | **0.028** | **0.49** |

### References

Burke, T. G., Gao, X 1994, Stabilization of topotecan in low pH liposomes composed of distearoylphosphatidylcholine, J. Parm. Sci. 83, 967-9
Emerson, D. L., et al., 2001 Antitumor efficacy, pharmacokinetics, and biodistribution of NX 211: a low-clearance liposomal formulation of lurtotecan, Clin. Canc. Res. 6, 2903-12.
Hertzberg, R. P. et al., 1989 Modification of the hydroxy lactone ring of camptothecin: inhibition of mammalian topoisomerase I and biological activity, J. Med. Cem. 3, 715-20.
Hsiang YH, Liu LF. Identification of mammalian DNA topoisomerase I as an intracellular target of the anticancer drug camptothecin. Cancer Res 1988;48(7):1722-6.
Liu, X. L. et al., A versatile prodrug approach for liposomal core-loading of water-insoluble camptothecin anticancer, Journal of the American Chemical Society 124, 7650-51
Moertel, C. G., Schutt, A. J., Reitemeier, R. J., Hahn, R. G., 1972, Phase II Study of Camptothecin (NSC-100880) in the treatment of gastrointestinal cancer; Cancer Chemother Rep) 96-101
Muggia, F. M., Creaven, P. J., Hansen, H. H., Cohen, M. H., Selawry, O. S., 1972, Phase I clinical trial of weekly and daily treatment with camptothecin (NSC-100880): correlation with preclinical studies 56, 515-21.
Schwarz C, Mehnert W. Freeze-drying of drug-free and drug-loaded solid lipid nanoparticles (SLN). Int J Pharm 1997;157(2):171-179.
Wall ME, Wani MC. Camptothecin and taxol: from discovery to clinic. J Ethnopharmacol 1996;51 (1-3):239-53; discussion 253-4.
Zunino F., Dallavalleb S., Laccaburea D, Berettaa G, Merlinib L, Pratesi G. Current status and perspectives in the development of camptothecins. Curr Pharm Ds. 2002; 8(27):2505-20.

## Claims

1. A method of producing a colloidal preparation comprising cationic colloidal nanoparticles and a camptothecin drug in the carboxylate form, wherein said preparation is substantially free of camptothecin lactone, comprising the steps of
d) providing a camptothecin drug in the carboxylate form,
e) providing drug free cationic nanoparticles and
f) incubating said camptothecin drug of step a) with the drug free cationic colloidal nanoparticles of step b) in an aqueous medium for a period of time sufficient to cause loading of said camptothecin drug into said cationic nanoparticles.

2. The method of claim 1, wherein said preparation is obtained after c) and which is suitable for immediately, e. g. directly administering it to a subject in need thereof.

3. The method of claim 1 or 2, wherein said cationic nanoparticles of step b) are selected from micelles, liposomes and nanocapsules.

4. The method of any one of the claims 1 to 3, wherein said camptothecin drug of step a) can be present as an aqueous solution or a solid product.

5. The method of any one of the claims 1 to 4, wherein said drug free cationic nanoparticles of step b) can be present as an aqueous dispersion or a solid product.

6. The method of any one of the claims 1 to 5, wherein said colloidal preparation has a pH in the range of about 6 to about 8.

7. The method of any one of the claims 1 to 6, wherein said camptothecin drug of step a) is selected from camptothecin, 10-OH-CPT or SN38.

8. The method of any one of the claims 1 to 7, wherein said cationic nanoparticles of step b) comprise cationic lipids, particularly cationic lipids selected from DOTAP or DMTAP.

9. The method of any one of the claims 1 to 8, wherein said incubation time of step c) is between about 10 min and about 6 hours, preferably between about 30 min and about 2 hours.

10. The method of any one of the claims 1 to 9, wherein said incubation temperature of step c) is between about 4°C and about 25°C, preferably about 25°C.

11. Kit comprising a) a camptothecin drug in the carboxylate form, b) drug freee cationic nanoparticles and optionally c) an aqueous medium, wherein the components a), b) and optionally c) are in separate containers.

12. The kit of claim 11 for the manufacture of a pharmaceutical compostion.

13. The kit of claim 1 to 2 for the manufacture of a medicament to treat an angiogenesis associated disease such as cancer.
